# EUROPEAN PATENT APPLICATION

(11) **EP 4 141 043 A1**
(43) Date of publication of application: **01.03.2023**
(21) Application number: 21193246.2
(22) Date of filing: 26.08.2021
(51) Int. Cl.: C08G 63/08, C07C 45/48, C07C 51/373, C08G 63/78, C08G 63/89, C08J 11/02

(54) **VALORISATION OF D-LACTIC ACID STREAM IN THE PRODUCTION PROCESS OF L-POLYLACTIC ACID**

(71) Applicant: Futerro S.A., 7760 Escanaffles (BE)
(72) Inventor: COSZACH, Philippe, 7760 Escanaffles (BE); VAN GANSBERGHE, Martin, 7760 Escanaffles (BE)
(74) Representative: AWA Benelux

(57) **Abstract**

The present invention discloses a valorisation method of a flux containing undesired D-lactic acid (ester(s)) in the production process of L-polylactic acid, the production thereof comprising: oligomerisation (30) of a substantially pure L-lactic acid feed; cyclisation (40) of the lactic acid oligomers, thereby obtaining lactides and a first residual stream transferred, at least partially, to a transesterification (80) and a hydrolysis (90) step; lactide purification (50), thereby obtaining purified lactides comprising L-lactide and meso-lactide, a second and a third residual stream, wherein the second residual stream is transferred, at least partially, to the oligomerisation (30) step and the third residual stream is transferred, at least partially, to a transesterification (80) and a hydrolysis step (90); polymerisation (60) of the purified lactides into polylactic acid; purification (70) of the polylactic acid, thereby obtaining purified polylactic acid comprising substantially L-polylactic acid and unreacted L-lactide, unreacted meso-lactide and impurities transferred, at least partially, to the lactide purification step (50); transesterification (80) and hydrolysis (90) of the at least partially transferred first and third residual streams, thereby obtaining a fourth and a fifth residual stream which are used, at least partially, as a base for the synthesis of molecules insensitive to the optical isometry D or L of lactic acid (esters).

## Description

### Technical Field

The present invention relates to a method of valorisation of a flux containing undesired D-lactic acid and/or undesired D-lactic acid ester(s) in the production process of L-polylactic acid. In particular, the present invention relates to a method of valorisation wherein a flux containing undesired D-lactic acid and/or undesired D-lactic acid ester(s) is used as a base for the synthesis of molecules insensitive to the optical isometry D or L of lactic acid and/or of lactic acid ester(s).

### Background Art

The last decades, biodegradable polymers have gained worldwide interest because of their capability to decompose in a natural environment. Examples of biodegradable polymers of interest are, without being limited thereto, aliphatic polyesters such as polylactic acid, polyhydroxybutyrate and polycaprolactone. In particular polylactic acid (PLA) has been studied because, next to being biodegradable, it is produced from lactic acid, which can be obtained from a raw material derived from a living body or a derivative thereof, such as fermented plant starch such as from corn, cassava, sugarcane or sugar beet pulp. Hence, PLA is considered as a high-safety and environmentally friendly polymer material. Nowadays, PLA can be found in various applications and forms, such as (stretched) films, fibres, injection molded products and filaments for 3D-printing.

Two main methods for the production of PLA from lactic acid are known. The first method is a direct polymerisation by polycondensation of lactic acid. Disadvantages of this method are the need for a solvent and the difficulties encountered in removing the water produced upon polycondensation out of the reaction medium.

The second method is a multi-step process, comprising oligomerisation of lactic acid, cyclisation of the oligomers into lactide, and polymerisation by ring opening of the lactide, thereby obtaining polylactic acid. One of the disadvantages of this method is that in each step by-products are obtained. Consequently, the efficiency of the polylactic acid production process is estimated to be around 50 mol%.

However, several of these by-products can be recycled, in particular by reuse in the PLA production process.

WO95/09879 discloses a process for the continuous production of substantially purified lactide and lactide polymers from lactic acid or an ester of lactic acid, including the steps of forming crude polylactic acid, preferably in the presence of a catalyst means in the case of the ester of lactic acid, to form a condensation reaction by-product and polylactic acid, and depolymerizing the polylactic acid in a lactide reactor to form crude lactide, followed by subsequent purification of the crude lactide in a distillation system, wherein the purified lactide can be a meso-lactide depleted flow of L-lactide and D-lactide. A purified lactide is then polymerized to form lactide polymers. By-products of the various reaction steps can be recycled.

With processes such as the one disclosed in WO95/09879, a total efficiency of about 75 mol% can be obtained. However, an efficiency of 75 mol% is considered insufficient for rendering the process applicable on industrial scale.

WO2015/086613 discloses a process wherein several by-products of the PLA production via the method comprising oligomerisation, cyclisation and polymerisation by ring opening, are reused in the PLA synthesis. Recycled by-products are in particular unreacted lactic acid, low weight and/or unreacted oligomers and lactide are reused. Part of the by-products is converted into lactic acid prior to reuse, by means of steps of transesterification, distillation and hydrolysis.

WO2014/180836 discloses a lactide purification process for use in for example the synthesis of L-polylactic acid. A purified lactide stream comprising L-lactide and meso-lactide is obtained from a crude lactide comprising L-lactide, D-lactide and meso-lactide. The lactide purification process comprises a first distillation step, a melt crystallization step and a second distillation step. The lactide purification process allows to reduce the amount of unwanted by-products due to the recovery of a large portion of the L-lactide and at least part of the meso-lactide in the crude lactide.

Lactic acid has two optically active enantiomers, D-lactic acid (or R-lactic acid) and L-lactic acid (or S-lactic acid). Consequently, three stereoisomers of lactide can be produced, namely L-lactide, D-lactide, and meso-lactide. The three stereoisomers of lactide can result in three stereochemical forms of polylactic acid (PLA), namely: D-PLA, L-PLA and a racemic mixture called D,L-PLA. The three stereochemical forms of polylactic acid comprise different properties. It is for example known that the presence of D-enantiomers in L-PLA results in a decrease of the glass transition temperature.

It is known that control of the physical properties of PLA can be achieved by adjusting the proportion of D-isomers and L- isomers. Consequently, an amorphous PLA can be obtained by adding an amount of D-enantiomer higher than 7 mol%. When the amount of D-enantiomer is lower than 7 mol%, the resulting PLA is considered semicrystalline. The degree of crystallization of PLA affects its melting temperature (Tm), glass transition temperature (Tg), and its mechanical properties, including elastic modulus and mechanical strength. In particular, and as is known, when the amount of D-enantiomers in L-PLA increases, both the Tm and Tg decrease.

Depending on the application of the polylactic acid, PLA comprising a high amount of one of the stereochemical forms is preferably used, or rather a mixture having specific amounts or ratios of the enantiomers. State of the art methods allow to obtain rather "pure" polylactic acid, i.e. polylactic acid substantially comprising L-polylactic acid and small amounts (e.g. 4% by weight or less, based on the total weight of PLA) of D,L-PLA. However, these methods generate several residual streams comprising compounds that are considered waste, thereby limiting the overall efficiency or the overall yield of the process.

In particular, in the production of L-polylactic acid, the residual streams comprise substantial amounts of compounds having a D-enantiometry (optical isometry), such as D-lactic acid, D-lactic acid oligomers, D-lactides, D,L-lactic acid oligomers, meso-lactides and D,L-PLA.

Hence, there is a need for the valorisation of these unwanted by-products. By valorising at least part of the unwanted residual products, less residual products are considered waste, hence, the efficiency of the production of L-PLA is increased.

### Summary of the Invention

It is an object of the present invention to overcome one or more of the above drawbacks. It is an aim of the invention to provide a valorisation of undesired by-products containing compounds comprising the optical isometry D, in particular D-lactic acid and D-lactic acid esters.

According to an aspect of the invention, there is provided a method of valorisation of a flux containing undesired D-lactic acid and/or one or more undesired D-lactic acid esters in the production process of L-polylactic acid as disclosed in the appended claims. The production of L-polylactic acid comprises the steps of:
i. Oligomerisation of a substantially pure L-lactic acid feed;
ii. Cyclisation of the lactic acid oligomers, thereby obtaining lactides and a first residual stream, wherein the first residual stream comprises unreacted oligomers, impurities and catalytic residues transferred, at least partially, to a transesterification and a hydrolysis step;
iii. Purification of the lactides, thereby obtaining purified lactides, a second and a third residual stream, wherein the purified lactides comprise L-lactide and meso-lactide and wherein the second residual stream comprises unreacted lactic acid and oligomers transferred, at least partially, to the oligomerisation step, and wherein the third residual stream comprises unreacted lactic acid, oligomers, impurities and residual L-lactide, D-lactide and meso-lactide transferred, at least partially, to a transesterification and a hydrolysis step;
iv. Polymerisation by ring opening of the purified lactides, thereby obtaining polylactic acid;
v. Purification of the polylactic acid, preferably by devolatilization, thereby obtaining purified polylactic acid comprising substantially L-polylactic acid and unreacted L-lactide, unreacted meso-lactide and impurities transferred, at least partially, to the lactide purification step;
vi. Transesterification and hydrolysis of the at least partially transferred first and third residual streams, thereby obtaining a fourth and a fifth residual stream, wherein the fourth residual stream comprises undesired D-lactic acid and wherein the fifth residual stream comprises one or more undesired D-lactic acid esters.

At least one, and preferably all, of the lactic acid of the fourth residual stream and the one or more lactic acid esters of the fifth residual stream is used, at least partially, as a base for the synthesis of molecules insensitive to the optical isometry D or L of lactic acid and/or lactic acid ester(s).

Advantageously, such molecules insensitive to the optical isometry D or L are selected from the group consisting of acrylic acid, acrylic ester, acetaldehyde, 2,3-pentanedione, pyruvic acid, and 1,2-propanediol.

The base for the synthesis of such molecules can comprise up to 100% of lactic acid and/or lactic acid esters obtained as residual product or by-product in the production of L-polylactic acid.

Advantageously, the substantially pure L-lactic acid feed comprises at least 90 % by weight of L-lactic acid based on the total weight of the lactic acid, preferably at least 95 % by weight, more preferably at least 98 % by weight, such as at least 99 % by weight or at least 99.5 % by weight.

Advantageously, the first residual stream comprises D-lactic acid oligomers and/or D,L-lactic acid oligomers.

Advantageously, the purified PLA can comprise up to 15% by weight of D,L-polylactic acid based on the total weight of the purified polylactic acid, such as up to 12% by weight, up to 10% by weight, or up to 4% by weight based on the total weight of the purified PLA. Depending on the applications for which the PLA is used, a larger or smaller concentration of D,L-PLA can be accepted wherein the properties, such as the crystallinity, Tg and Tm, of the obtained PLA remain within acceptable ranges.

Advantageously, unreacted L-lactide, unreacted meso-lactide and impurities are obtained as well, which are, preferably, transferred, at least partially, to the lactide purification step. Further, part of the unreacted L-lactide, the unreacted meso-lactide and the impurities can be transferred to the transesterification and the hydrolysis step.

Advantageously, the lactide purification step comprises one or more sub-steps. The sub-steps can comprise a pre-purification step, a melt purification step and a separation step.

Advantageously, part of the second residual stream is transferred to the transesterification and/or the hydrolysis step.

Advantageously, the method further comprises a distillation step between the transesterification step and the hydrolysis step. Advantageously, the fifth residual stream is obtained at the distillation step. Advantageously, the fourth residual stream is obtained at the hydrolysis step.

Preferably, the fourth residual stream comprises between 2% and 50% by weight of D-lactic acid based on the total weight of the fourth residual stream, for example between 2.5% and 40% by weight, preferably between 4% and 30% by weight, such as between 5% and 25% by weight, more preferably between 10% and 20% by weight.

Preferably, the fifth residual stream comprises between 2% and 50% by weight of D-lactic acid esters based on the total weight of the fifth residual stream, for example between 5% and 45% by weight, preferably between 10% and 40% by weight, more preferably between 15% and 35% by weight.

The inventors have noticed that by means of the methods of the invention, the efficiency and the flexibility of the production of L-PLA can be increased significantly compared to known processes comprising recycling of (part of) the by-products. With "production flexibility of the L-PLA" it is meant in the present invention the capability to obtain L-PLA having the requested or required properties, such as Tg and Tm, and thus wherein the ratio of L-PLA and D,L-PLA can be adapted over a wide range to obtain L-PLA having the optimal properties for a given application or use.

In particular, methods of the invention for the production of L-polylactic acid provide for a limited re-introduction of the residual streams comprising substantial amounts of compounds having a D-enantiometry and a D,L-enantiometry. Since these D-compounds, and to a smaller extent these D,L-compounds, contribute to the production of D,L-polylactic acid, they will impact the final properties of the obtained (L-)polylactic acid. Hence, their limited re-introduction in the production process of L-polylactic acid reduces the impact on the properties of the obtained L-PLA, and even renders the impact almost nihil. Further, methods of the invention provide for recycling or reuse of the remaining portion of the residual streams, so that the overall efficiency is increased and thus the amount of waste products is reduced when compared to prior art methods. Consequently, the invention provides methods that can be used and implemented on an industrial scale.

### Brief Description of Drawings

Aspects of the invention will now be described in more detail with reference to the appended drawings, wherein same reference numerals illustrate same features.
Figure 1 represents schematically the process steps of methods of the invention.
Figure 2 represents schematically a lactide purification step of methods of the invention.

### Detailed description

The present invention will now be described more fully hereinafter with reference to the accompanying drawings, in which embodiments of the invention are shown. This invention may, however, be embodied in many different forms and should not be construed as limited to the embodiments set forth herein; rather, these embodiments are provided for thoroughness and completeness.

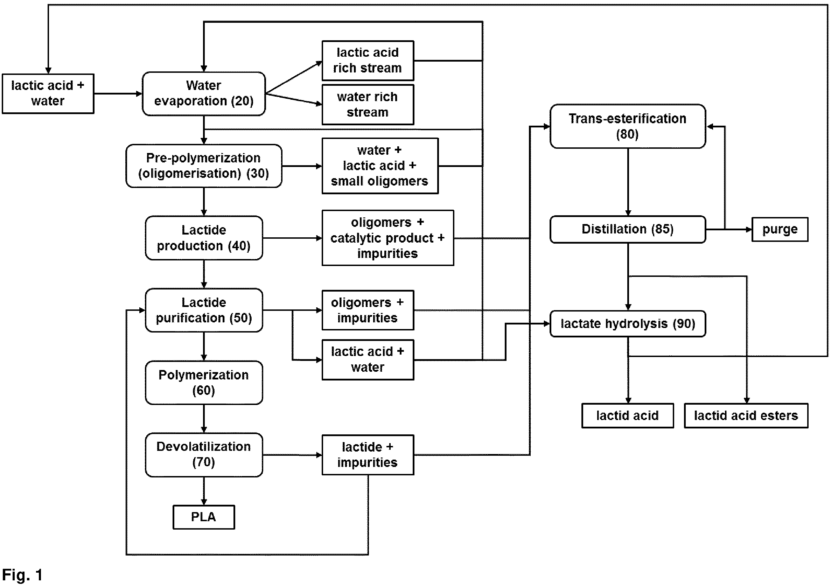

Referring to Figure 1, a substantially pure L-lactic acid feed is provided. The feed can comprise a limited amount of D-lactic acid, such as at most 5% by weight based on the total weight of the lactic acid, such as at most 4% by weight, at most 3% by weight, at most 2% by weight, at most 1.5% by weight, at most 1% by weight, or at most 0.5% by weight of D-lactic acid.

The substantially pure L-lactic acid feed can be provided as an aqueous solution, wherein the solution comprises typically between 50% and 100% by weight of lactic acid based on the total weight of the aqueous solution. When the L-lactic acid is provided as an aqueous solution, the water is advantageously evaporated in an evaporation 20 step (as shown in Fig. 1).

The L-lactic acid is then pre-polymerised by oligomerisation 30, thereby obtaining lactic acid oligomers. A catalyst can optionally be used in the oligomerisation step 30, such as a tin based catalyst. The oligomerisation 30 is advantageously performed in one or more steps at a temperature between 90°C and 250°C, preferably between 100°C and 200°C, and at a pressure between 2.5 mbar and 1000 mbar, preferably between 5 mbar and 500 mbar.

The oligomers are generally of low viscosity and of low molecular weight, such as between 400 Dalton and 5000 Dalton, as measured by chromatography by gel permeation compared to standard polystyrene in chloroform at 30°C.

The reaction product of the oligomerisation 30 step can further comprise unreacted lactic acid, water and small lactic acid oligomers. Any unreacted lactic acid, water and small lactic acid oligomers is advantageously transferred, at least partially, back to the oligomerisation 30 step, and water is optionally evaporated.

Hence, the lactic acid that is pre-polymerised can comprise a mixture of the substantially pure L-lactic acid feed and recycled lactic acid, wherein the recycled lactic acid comprises unreacted lactic acid of a previous pre-polymerisation step 30. The lactic acid can comprise up to 50% by weight of recycled lactic acid, based on the total weight of the lactic acid, such as up to 40% by weight, up to 30% by weight, up to 20% by weight, up to 10% by weight, or up to 5% by weight, based on the total weight of the lactic acid.

The lactic acid oligomers are then converted in lactides by means of cyclisation (cyclisation 40 step) in the presence of a catalyst. The catalyst can be any catalyst known in the art for the cyclisation of lactic acid oligomers into lactides. A particular example of a suited catalyst is a tin based catalyst.

The cyclisation 40 is advantageously performed at a temperature between 100°C and 350°C, such as between 150°C and 320°C, and at a pressure between 1 mbar to 500 mbar, such as between 2 mbar and 250 mbar, between 3 mbar and 100 mbar, preferably between 5 mbar and 80 mbar.

A first residual stream is obtained as well upon the cyclisation of the lactic acid oligomers. The first residual stream can comprise unreacted lactic acid oligomers, impurities (such as non-volatile impurities) and catalytic residues. The first residual stream can further comprise low molecular weight polylactic acid, i.e. polylactic acid having a molecular weight between 2000 Dalton and 8000 Dalton, as measured by chromatography by gel permeation compared to standard polystyrene in chloroform at 30°C. Advantageously, unreacted lactic acid oligomers in the first residual stream comprise D-lactic acid oligomers and/or D,L-lactic acid oligomers.

The first residual stream is advantageously transferred, at least partially, to a transesterification 80 and a hydrolysis 90 step. Optionally, part of the first residual stream can be transferred to the pre-polymerisation step 30.

The lactides obtained in the cyclisation 40 step comprise a mixture of L-lactide, D-lactide, meso-lactide and impurities. Therefore, the lactides are purified, preferably chemically and enantiomerically. During the lactide purification 50 step, the D-lactide, L-lactide, meso-lactide and impurities are separated. Optionally, part of the meso-lactide is then added to the L-lactide for the production of D,L-polylactic acid. Hence, purified lactides are obtained, wherein the purified lactides comprise L-lactide and meso-lactide. Further, a second residual stream and a third residual stream are obtained.

The second residual stream advantageously comprises unreacted lactic acid and oligomers, and can also comprise traces of water. Advantageously, the second residual stream is transferred, at least partially, to the oligomerisation 30 step. Optionally, at least part of the water comprised in the second residual stream can be evaporated prior to transferring the at least part of the second residual stream to the oligomerisation 30 step.

Further, part of the second residual stream can be transferred to a transesterification 80 step and/or a hydrolysis 90 step.

The third residual stream advantageously comprises unreacted L-lactic acid and/or unreacted D-lactic acid, oligomers, impurities and residual L-lactide, D-lactide and meso-lactide. Advantageously, the third residual stream is transferred, at least partially, to a transesterification 80 and a hydrolysis 90 step

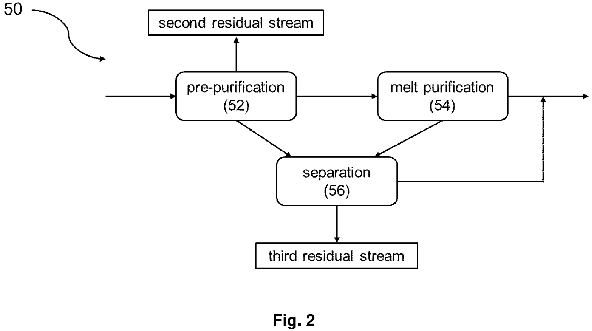

According to an embodiment of the invention, the lactide purification step can comprise one or more sub-steps, such as a pre-purification step, a melt purification step and a separation step. Figure 2 discloses such sub-steps of the lactide purification 50 step.

The lactide obtained in the cyclisation step 40 is pre-purified 52. Upon pre-purification 52, pre-purified L-lactide, a mixture comprising D-lactide, meso-lactide, unreacted lactic acid and/or unreacted oligomers and (traces of) water, and the second residual stream are obtained.

The pre-purified L-lactide can still comprise D-lactide, meso-lactide, unreacted oligomers and/or impurities, wherein the relative amount of D-lactide and meso-lactide is lower than in the lactide prior to pre-purification. The pre-purified L-lactide is advantageously transferred, at least partially, to a melt-purification step 54. When no melt-purification step is carried out (not shown), the pre-purified L-lactide is advantageously transferred, at least partially, to the polymerisation step 60.

The mixture obtained in the pre-purification step 52 comprising D-lactide, meso-lactide, unreacted lactic acid and/or unreacted oligomers can still comprise traces of L-lactide. The mixture is advantageously transferred, at least partially, to a separation step 56. When no separation step is carried out (not shown), the mixture is advantageously transferred, at least partially, to a transesterification 80 and a hydrolysis 90 step.

Next, the pre-purified L-lactide is further purified in a melt purification step 54. When no pre-purification step 52 is carried out (not shown), the lactide is purified in the melt purification step 54. Upon melt purification, purified L-lactide and a mixture comprising D-lactide, meso-lactide, unreacted oligomers and/or impurities are obtained.

The purified L-lactide is advantageously transferred, at least partially, to the polymerisation step 60.

The mixture obtained in the melt purification step 54 comprising D-lactide, meso-lactide, unreacted oligomers and/or impurities oligomers can still comprise traces of L-lactide. The mixture is advantageously transferred, at least partially, to a separation step 56. When no separation step is carried out (not shown), the mixture is advantageously transferred, at least partially, to a transesterification 80 and a hydrolysis 90 step.

The mixtures obtained in the pre-purification step 52 and in the melt purification step 54 are then further purified in a separation step 56, wherein a purified lactide mixture comprising L-lactide and meso-lactide, and the third residual stream are obtained. Advantageously, the purified lactide mixture has a residual acidity equal to or lower than 50 mEq/kg, preferably equal to or lower than 30 mEq/kg, more preferably equal to or lower than 20 mEq/kg, and even more preferably equal to or lower than 10 mEq/kg. A low residual acidity indicates the presence of a minimal amount of impurities, which leads to a more efficient polymerization of the lactides.

Further, the purified lactide mixture comprising L-lactide and meso-lactide advantageously has a water content equal to or lower than 150 ppm, preferably lower than 125 ppm, more preferably equal to or lower than 100 ppm.

The purified lactide mixture comprising L-lactide and meso-lactide is transferred, at least partially, to the polymerisation step 60.

Referring back to Figure 1, the purified lactides, advantageously comprising L-lactide and meso-lactide, are polymerized to polylactic acid in a polymerization 60 step. The polymerization is a polymerization by ring opening.

Advantageously, the polylactic acid has a molecular weight between 10000 Dalton and 200000 Dalton, as measured by chromatography by gel permeation compared to standard polystyrene in chloroform at 30°C.

Advantageously, the polylactic acid comprises substantially L-polylactic acid, but can also comprise D,L-polylactic acid due of the presence of meso-lactide in the purified lactides. Further, the reaction product of the polymerization 60 step can comprise unreacted L-lactide and, in limited concentrations, unreacted meso-lactide and traces of impurities.

The polylactic acid reaction product can be purified in a PLA purification 70 step, thereby obtaining a stream of purified polylactic acid and a stream comprising unreacted L-lactide, unreacted meso-lactide and impurities. Preferably, the polylactic acid purification 70 step is a devolatilization step.

Advantageously, the purified PLA comprises substantially L-polylactic acid. The purified PLA can comprise up to 15% by weight of D,L-polylactic acid based on the total weight of the purified polylactic acid, such as up to 12% by weight, up to 10% by weight, up to 5% by weight, or up to 1% by weight, depending on the application for which the polylactic acid is used.

The stream comprising unreacted L-lactide, unreacted meso-lactide and impurities can be transferred, at least partially, to the lactide purification 50 step. Further, part of the stream can be transferred to a transesterification 80 and a hydrolysis 90 step.

Methods according to the invention further comprise transesterification 80 and hydrolysis 90 of the at least partially transferred first residual stream and of the at least partially transferred third residual streams. Upon transesterification 80 and hydrolysis 90 of at least part of the first and third residual streams, a fourth and a fifth residual stream are obtained.

Transesterification 80 comprises the reaction of lactic acid and lactic acid oligomers with alcohol, thereby obtaining alkyl lactate. Advantageously, the transesterification 80 step is performed according to processes known in the art and by means of known conditions and catalysts. Advantageously, the transesterification is performed at a temperature between 50°C and 250°C, preferably between 80°C and 200°C, more preferably between 100°C and 180°C, and at a pressure between 1 bar and 20 bar, preferably between 2 bar and 15 bar. At least part of the catalyst for the transesterification 80 step can be provided by means of the first residual stream.

Hydrolysis 90 comprises conversion of the alkyl lactate, with the aid of water, in lactic acid and alcohol, and optionally lactic acid derivatives. Advantageously, the hydrolysis 90 step is performed according to processes known in the art and by means of known conditions. Advantageously, the hydrolysis is performed at a temperature between 50°C and 200°C, preferably between 70°C and 180°C, more preferably between 90°C and 150°C, and at a pressure between 0.01 bar and 10 bar, preferably between 1 bar and 3 bar. The hydrolysis 90 step can be performed in the presence of a catalyst. The catalyst can be the lactic acid itself. The obtained alcohol is most often an aliphatic alcohol having from 1 to 12 carbon atoms. The alcohol can be withdrawn from the reaction medium in order to increase the reaction efficiency, according to methods known in the art.

Advantageously, the fourth residual stream comprises between 10% and 90% by weight of lactic acid based on the total weight of the fourth residual stream, such as between 20% and 80% by weight, preferably between 25% and 75% by weight, more preferably between 40% and 60% by weight.

The fourth residual stream can also comprise undesired D-lactic acid. Preferably, the lactic acid comprised in the fourth residual stream comprises between 2.5% and 80% by weight of undesired D-lactic acid based on the total weight of lactic acid, such as between 5% and 75% by weight, preferably between 10% and 60% by weight, more preferably between 15% and 40% by weight.

Preferably, the fourth residual stream comprises between 0.25% and 75% by weight of D-lactic acid based on the total weight of the fourth residual stream, for example between 0.5% and 70% by weight, between 1% and 60% by weight, between 2% and 50% by weight, preferably between 5% and 30% by weight, more preferably between 10% and 20% by weight, such as between 10% and 15% by weight.

Advantageously, the fifth residual stream, obtained at the transesterification 80 step, comprises between 0.1% and 90% by weight of one or more lactic acid esters based on the total weight of the fifth residual stream, such as between 0.5% and 70% by weight, between 1% and 60% by weight, preferably between 2% and 50% by weight, such as between 5% and 45% by weight, more preferably between 10% and 40% by weight, for example between 15% and 35% by weight.

Optionally, and as shown in Fig. 1, a distillation step 85 can be provided between the transesterification step 80 and the hydrolysis step 90. When the method comprises a distillation step 85, the fourth residual stream is advantageously obtained at the hydrolysis step 90 and/or the fifth residual stream is advantageously obtained at the distillation step 85.

The distillation 85 of the alkyl lactate obtained in the transesterification 80 step allows to separate the lactate molecules from heavier components, such as catalytic residues, oligomers and unreacted products. Advantageously, the distillation 85 step is performed at a pressure between 0.01 bar to 5 bar, preferably between 0.05 bar and 2.5 bar, more preferably between 0.1 bar and 1 bar, and at a temperature between 25°C and 200°C, preferably between 40°C and 180°C, more preferably between 60°C and 150°C. The lactate molecules are transferred, at least partially, to the hydrolysis 90 step. The heavier components are transferred, at least partially, back to the transesterification 80 step. Optionally, part of the heavier components can for example be purged and optionally be treated, for example by means of filtration or decantation, to separate for example the catalytic residues from the oligomers. Optionally, the catalytic residues can be transferred, at least partially, to the transesterification 80 step, the oligomerisation 30 step, and/or the cyclisation 40 step as such or after an additional treatment such as drying.

Advantageously, the fifth residual stream comprises one or more undesired D-lactic acid esters. Preferably, the lactic acid esters comprised in the fifth residual stream comprises between 2.5% and 80% by weight of undesired D-lactic acid esters based on the total weight of lactic acid esters, such as between 5% and 60% by weight, more preferably between 10% and 40% by weight.

Preferably, the fifth residual stream comprises between 0.25% and 75% by weight of one or more D-lactic acid esters based on the total weight of the fifth residual stream, for example between 1% and 60% by weight, preferably between 2% and 50% by weight, such as between 5% and 45% by weight, more preferably between 10% and 40% by weight, for example between 15% and 35% by weight.

Advantageously, the lactic acid of the fourth residual stream and/or the one or more lactic acid esters of the fifth residual stream are used, at least partially, as a base for the synthesis of molecules insensitive to the optical isometry D or L of lactic acid and/or lactic acid ester(s). Preferably, the lactic acid of the fourth residual stream and the one or more lactic acid esters of the fifth residual stream are used, at least partially, as such a base.

Preferably, such molecules insensitive to the optical isometry D or L are selected from the group consisting of acrylic acid, acrylic ester, acetaldehyde, 2,3-pentanedione, pyruvic acid, and 1,2-propanediol.

The base for the synthesis of such molecules can comprise up to 100% of lactic acid and/or lactic acid esters obtained as residual product or by-product in the production of L-polylactic acid. For example, said molecules insensitive to the optical isometry D or L can be synthesised from a mixture of such residual lactic acid (esters) and lactic acid (esters) obtained from another source, such as "virgin" lactic acid obtained by fermentation of (plant) starch. Preferably, the lactic acid used for the synthesis of such molecules comprises between 10% and 100% by weight of residual lactic acid (esters) based on the total weight of lactic acid (esters) used in the synthesis, such as at least 10% by weight, at least 20% by weight, preferably at least 25% by weight, more preferably at least 50% by weight, for example between 50% and 100% by weight.

### Examples

### Example 1

6 kg of an 88% aqueous solution of substantially pure L-lactic acid comprising 1% by weight of D-lactic acid based on the total weight of the lactic acid was subjected to water elimination 20. The water elimination comprised heating the solution at a temperature of 100°C and at a reduced pressure of 250 mbar. The recovered water was purged and the recovered L-lactic acid was sent to a pre-polymerisation reactor where it was pre-polymerized 30 by oligomerisation. The oligomerisation 30 was carried out at a temperature of 165°C and at a reduced pressure of 250 mbar and down to 80 mbar. The obtained oligomers of lactic acid had a molecular weight of about 950 Dalton (comprised between 900 and 1000 Dalton).

Water released during the pre-polymerisation 30 was withdrawn and purged. Any unreacted lactic acid was recovered and recycled back to the reactor for water elimination 20.

The lactic acid oligomers were then sent to a reactor for cyclisation 40. The cyclisation of the oligomers of lactic acid was performed in the presence of Sn octanoate as catalyst, and at a temperature of 250°C and at a pressure of 10 mbar, thereby producing a crude lactide stream.

A first residual stream comprising any unreacted oligomers, catalytic residues as well as the heavier components was withdrawn from the cyclisation reactor and was sent to a reactor for trans-esterification 80.

The crude lactide stream was sent to a lactide purification step 50. The lactide purification 50 comprised pre-purification 52 and melt crystallization 54 of the lactides, wherein a flow of purified lactides comprising L-lactide and meso-lactide was obtained, as well as a second and a third residual stream. The second residual stream comprised unreacted lactic acid and oligomers and was recycled back to the reactor for oligomerisation 30. The third residual stream comprised unreacted lactic acid, residual L-lactide, D-lactide and meso-lactide, oligomers, impurities and heavy components. The third residual stream was sent to a reactor for trans-esterification 80 and hydrolysis 90.

The purified lactides were then subjected during 30 minutes to ring opening polymerization 60 in the presence of Sn octanoate as catalyst and at a temperature of 185°C.

The obtained PLA was then purified 70 by devolatilization, thereby obtaining purified polylactic acid comprising substantially L-polylactic acid and unreacted lactides. Any unreacted lactides were recycled to the lactide purification 50. Any catalytic or other impurities were sent to a reactor for trans-esterification 80.

Hydrolysis 90 of the first and third residual streams resulted in a fourth residual stream comprising 25% by weight of D-lactic acid based on the total weight of the fourth residual stream. During this test, a portion of the fifth residual stream comprising 35% by weight of D-ethyl lactate based on the total weight of the fifth residual stream was collected at the transesterification 80.

The fourth and fifth residual stream were then used for the synthesis of pyruvic acid. First, the residual streams were transformed into ethyl lactate by a known process, then the feed comprising ethyl lactate was introduced at the top of a tubular reactor in order to be vaporized. The vapour was then passed through three layers of catalyst made of silver crystal beads. The reaction was carried out at a temperature of 550°C and at a pressure of 1.2 bar. The gaseous reaction mixture was then cooled down to 20°C and washed with water, and the ethyl pyruvate was recovered in good yield (72%). The ethyl pyruvate was of equivalent quality than an ethyl pyruvate produced from non-recycled ethyl lactate with a similar conversion and selectivity.

### Example 2

A 92% aqueous solution of substantially pure L-lactic acid comprising 0.8% by weight of D-lactic acid based on the total weight of the lactic acid was subjected to water elimination 20. The water elimination comprised heating the solution at a temperature of 100°C and at a reduced pressure of 250 mbar.

The L-lactic acid was then pre-polymerised by oligomerisation 30 at a temperature of 160°C and at a reduced pressure of 250 mbar. The pre-polymerised lactic acid comprised a mixture of a substantially pure L-lactic acid feed and 5% by weight of recycled lactic acid, based on the total weight of the lactic acid.

The reaction product of the oligomerisation step 30 further comprised unreacted lactic acid, water and small lactic acid oligomers, which were transferred back to the reactor for oligomerisation 30.

The lactic acid oligomers were then sent to a cyclisation step 40. The cyclisation of the oligomers of lactic acid was performed in the presence of Sn octanoate as catalyst, and at a temperature of 250°C and at a pressure of 10 mbar, thereby producing a crude lactide stream.

A first residual stream comprising unreacted D-lactic acid oligomers, unreacted D,L-lactic acid oligomers and catalytic residues was withdrawn from the cyclisation reactor and was sent to a reactor for trans-esterification 80.

The crude lactide stream was sent to a lactide purification step 50. The lactide purification 50 comprised pre-purification 52 and melt crystallization 54 of the lactides, wherein a flow of purified lactides comprising L-lactide and meso-lactide was obtained, as well as a second and a third residual stream. The second residual stream comprised unreacted lactic acid and oligomers and was recycled back to the reactor for oligomerisation 30. The third residual stream comprised unreacted lactic acid, oligomers, impurities and heavy components, the heavy components comprising residual L-lactide, D-lactide and meso-lactide. The third residual stream was sent to a reactor for trans-esterification 80 and hydrolysis 90.

The purified lactides were then subjected during 30 minutes to ring opening polymerization 60 in the presence of Sn octanoate as catalyst and at a temperature of 185°C.

The obtained PLA was then purified 70 by devolatilization, thereby obtaining purified polylactic acid comprising substantially L-polylactic acid and unreacted lactides. Any unreacted lactides were recycled to the lactide purification 50. Any catalytic or other impurities were sent to a reactor for trans-esterification 80.

Hydrolysis 90 of the first and third residual streams resulted in a fourth residual stream comprising 15% by weight of D-lactic acid based on the total weight of the fourth residual stream. During this test, a portion of the fifth residual stream comprising 20% of D-ethyl lactate based on the total weight of the fifth residual stream was collected at the transesterification 80.

The fourth and the fifth residual streams were then used for the synthesis of 1,2-propanediol via a catalytic hydrogenation by using a ruthenium catalyst deposited on a microporous carbon support in an amount of a Ru / C ratio of 5% by weight.

First, a 10% aqueous lactic acid solution was prepared with the fourth and fifth residual streams and was introduced into a CSTR reactor. The hydrogenation reaction was carried out at a H₂/ lactic acid ratio of 5/1 and at a temperature of 100°C and at a pressure of 8 MPa. The 1,2-propanediol was then recovered in good yield (85%). The 1,2-propanediol was of equivalent quality than a 1,2-propanediol produced from non-recycled lactic acid with similar conversion and selectivity.

### Example 3

The same PLA production steps as disclosed in example 2 were carried out, resulting in substantially pure L-PLA and a fourth and a fifth residual stream.

The fourth residual stream comprising 10% of D-lactic acid based on the total weight of the fourth residual stream was used to prepare 2,3-pentanedione by condensation of this recycled D-lactic acid, in the presence of a sodium phosphate catalyst deposited on a silica/alumina support.

A 50% aqueous solution of lactic acid was prepared with the fourth residual stream which was brought into contact with the catalyst described above at a temperature of 300°C and under a pressure of 5 MPa.

2,3-pentanedione was obtained in good yield (76%), it was of equivalent quality than a 2,3-pentanedione produced from non-recycled lactic acid with similar conversion and selectivity.

## Claims

1. Method of valorisation of a flux containing undesired D-lactic acid and/or one or more undesired D-lactic acid esters in the production process of L-polylactic acid, said process comprising the steps of:
i. Oligomerisation (30) of a substantially pure L-lactic acid feed;
ii. Cyclisation (40) of the lactic acid oligomers, thereby obtaining lactides and a first residual stream, wherein the first residual stream comprises unreacted oligomers, impurities and catalytic residues transferred, at least partially, to a transesterification (80) and a hydrolysis (90) step;
iii. Purification (50) of the lactides, thereby obtaining purified lactides, a second and a third residual stream, wherein the purified lactides comprise L-lactide and meso-lactide and wherein the second residual stream comprises unreacted lactic acid and oligomers transferred, at least partially, to the oligomerisation (30) step, and wherein the third residual stream comprises unreacted lactic acid, oligomers, impurities and residual L-lactide, D-lactide and meso-lactide transferred, at least partially, to a transesterification (80) and a hydrolysis step (90);
iv. Polymerisation (60) by ring opening of the purified lactides, thereby obtaining polylactic acid;
v. Purification (70) of the polylactic acid, preferably by devolatilization, thereby obtaining purified polylactic acid comprising substantially L-polylactic acid and unreacted L-lactide, unreacted meso-lactide and impurities transferred, at least partially, to the lactide purification step (50);
vi. Transesterification (80) and hydrolysis (90) of the at least partially transferred first and third residual streams, thereby obtaining a fourth and a fifth residual stream, wherein the fourth residual stream comprises undesired D-lactic acid and wherein the fifth residual stream comprises one or more undesired D-lactic acid esters;
wherein the lactic acid of the fourth residual stream and the one or more lactic acid esters of the fifth residual stream are used, at least partially, as a base for the synthesis of molecules insensitive to the optical isometry D or L of lactic acid and/or lactic acid ester(s), and in particular molecules selected from the group consisting of acrylic acid, acrylic ester, acetaldehyde, 2,3-pentanedione, pyruvic acid, and 1,2-propanediol.

2. Method according to claim 1, wherein the first residual stream substantially comprises D-lactic acid oligomers and/or D,L-lactic acid oligomers.

3. Method according to claim 1 or claim 2, wherein the purified polylactic acid comprises up to 15% by weight of D,L-polylactic acid based on the total weight of the purified polylactic acid.

4. Method according to any one of the preceding claims, wherein the lactide purification (50) step comprises one or more sub-steps, wherein the sub-steps comprise a pre-purification (52) step, a melt purification (54) step and a separation (56) step.

5. Method according to any one of the preceding claims, wherein part of the unreacted L-lactide, the unreacted meso-lactide and the impurities are transferred to the transesterification (80) and the hydrolysis (90) step.

6. Method according to any one of the preceding claims, wherein part of the second residual stream is transferred to the transesterification (80) and/or the hydrolysis step (90).

7. Method according to any one of the preceding claims, further comprising a distillation step (85) between the transesterification (80) step and the hydrolysis (90) step.

8. Method according to claim 7, wherein the fifth residual stream is obtained at the distillation step (85).

9. Method according to any one of the preceding claims, wherein the fourth residual stream is obtained at the hydrolysis step (90).

10. Method according to any one of the preceding claims, wherein the fourth residual stream comprises between 2% and 50% by weight of D-lactic acid based on the total weight of the fourth residual stream.

11. Method according to any one of the preceding claims, wherein the fourth residual stream comprises between 5% and 30% by weight of D-lactic acid based on the total weight of the fourth residual stream.

12. Method according to any one of the preceding claims, wherein the fourth residual stream comprises between 10% and 20% by weight of D-lactic acid based on the total weight of the fourth residual stream.

13. Method according to any one of the preceding claims, wherein the fifth residual stream comprises between 2% and 50% by weight of D-lactic acid ester based on the total weight of the fifth residual stream.

14. Method according to any one of the preceding claims, wherein the fifth residual stream comprises between 10% and 40% by weight of D-lactic acid ester based on the total weight of the fifth residual stream.
